# EUROPEAN PATENT APPLICATION

(11) **EP 1 374 916 A1**
(43) Date of publication of application: **02.01.2004**
(21) Application number: 03014124.6
(22) Date of filing: 24.06.2003
(51) Int. Cl.: A61L 2/26, A61L 2/07, A61L 2/16

(54) **Autoclave base and shelf**

(30) Priority: 27.06.2002 US 183238
(71) Applicant: Coltene/Whaledent, Inc., Mahwah, New Jersey 07430 (US)
(72) Inventor: Sullivan, Jerry, Ridgewood, NJ 07450 (US)
(74) Representative: Grättinger & Partner (GbR)

(57) **Abstract**

A support base for a front-loading sterilizer includes a retractable shelf for staging items to be loaded into the sterilizer, and for drying and cooling items that are removed from the sterilizer. The retractable shelf includes a holding element characterized by periodically-spaced lateral troughs and crests. Items to be dried may be positioned on the crests so that ambient air may flow below the items for drying and condensate may drain into the troughs. The drying element may be fixed to or removable from the holding element. The support base may optionally and integrally include chassis members of the sterilizer.

## Description

### FIELD OF THE INVENTION

This invention relates to a support base and work surface used in conjunction with industrial apparatus. More particularly, the invention relates to a sterilizer support base having a retractable shelf.

### BACKGROUND OF THE INVENTION

Steam autoclaving is a well-known and reliable method used widely to sterilize media and lab equipment as well as to decontaminate infectious waste. Autoclaves introduce saturated steam within a pressurized chamber (for example, at pressures of 15 psi or more) in order to generate sufficient temperatures (for example, at or above 275 degrees Fahrenheit) to achieve sterilization. Heat produced by saturated steam at such temperatures is effective in damaging essential cellular structures of biological organisms present in the pressurized chamber, and with sufficient time, in killing such organisms.

Items placed in autoclave chambers are subjected to pressurized steam. Care is required in removing these items after the autoclaving process has completed. The chamber must be depressurized before opening. Upon removal, water condensate may be present on the sterilized items and require evaporation and/or draining. Also, the sterilized items may be slippery and difficult to handle. Accordingly, it would be desirable to provide an area adjacent to the autoclave and appropriately configured for receiving and drying sterilized items as they are removed from the autoclave chamber.

Devices for holding items placed within an autoclave are known in the art (for example, see U.S. Patent No. 4,670,227, issued to Smith on June 2, 1987). Specialized work surfaces have been developed for use with other appliances (for example, see U.S. Patent No. 4,436,356, issued to Stelling on March 13,1984). However, to date, there does not appear to be disclosed a specialized work surface positioned adjacent to an autoclave for receiving and drying sterilized items as they are removed from the autoclave chamber.

### SUMMARY OF THE INVENTION

A convenient work surface both for staging items to be placed in an autoclave for sterilizing and for retaining items removed from an autoclave for staging, cooling or drying is provided by a novel autoclave support base. The support base is equally well suited for application to other types of sterilizers such as dry-heat and chemical vapor sterilizers.

The support base comprises a base housing having a top surface for receiving and supporting the autoclave, and left and right support members respectively and downwardly attached in proximity to left and right opposing edges of the top surface, such that the top surface and left and right support members define a cavity in the base housing. A holding shelf is slidably mounted within the cavity in the base housing.

The holding shelf includes a holding surface depressedly positioned with respect to front, rear, left and right edges of the holding shelf so that surfaces defined between the front, rear, left and right edges of the holding shelf and the holding surface act to confine items placed on the holding surface. The holding surface may be pitched toward one or more perforations in the holding surface to facilitate drainage.

In an illustrative embodiment of the present invention, a holding element is positioned within the front, rear, left and right edges of the holding shelf and supported by the holding surface for holding the staged or removed items. A drying surface of the holding element has a plurality of periodically and laterally spaced troughs and crests, each crest defining an elevated positioning surface for holding drying items in order to promote a flow of ambient air under the items. The holding element may be fixed to the holding shelf, or may be removable. These crests may include indentations for positioning items on the crests of the holding element.

In another illustrative embodiment of the present invention, the support base includes at least one securing panel upwardly attached at an edge of the top surface for fastening to the autoclave. The at least one securing panel may include apertures for permitting access to the autoclave chamber and controls, and for fastening autoclave door hinge and catch hardware in a manner that effectively secures the securing panel to the autoclave. Preferably, the at least one securing panel comprises a chassis of the autoclave for holding components of the autoclave together in an assembly.

### BRIEF DESCRIPTION OF THE DRAWINGS

A more complete understanding of the invention may be obtained by reading the following description of specific illustrative embodiments of the invention in conjunction with the appended drawing in which:
FIG. 1 provides a perspective view of an inventive autoclave support base, with the holding shelf extended and as seen from a right front side;
FIG. 2 provides a perspective view of the support base of FIG. 1 as seen from a right rear side, and illustrating a first embodiment of a holding element positioned in the holding shelf;
FIG. 3 provides a perspective view of the support base of FIG. 1 as seen from the right front side, further depicting placement of an autoclave on the top surface of the support base;
FIG. 4 provides a front view of the support base of FIG. 1 further depicting placement of an autoclave on the top surface;
FIG. 5 illustrates an element of the present invention including a spring catch mechanism for retaining the holding shelf of the support base in a retracted position;
FIGs. 6 (a), 6(b) present an alternative shelf and holding element; and
FIG. 7 shows an alternative embodiment of the support base having an integral autoclave chassis defined by one or more base housing securing panels.

In the various figures, like reference numerals wherever possible designate like or similar elements of the invention.

### DETAILED DESCRIPTION OF THE PREFERRED EMBODIMENTS

The following detailed description includes a description of the best mode or modes of the invention presently contemplated. Such description is not intended to be understood in a limiting sense, but to be an example of the invention presented solely for illustration thereof, and by reference to which in connection with the following description and the accompanying drawing one skilled in the art may be advised of the advantages and construction of the invention. For example, while the description is made with reference to autoclave sterilizers, application to other types of sterilizers, such as dry-heat and chemical vapor sterilizers, is fully contemplated within the scope of the invention.

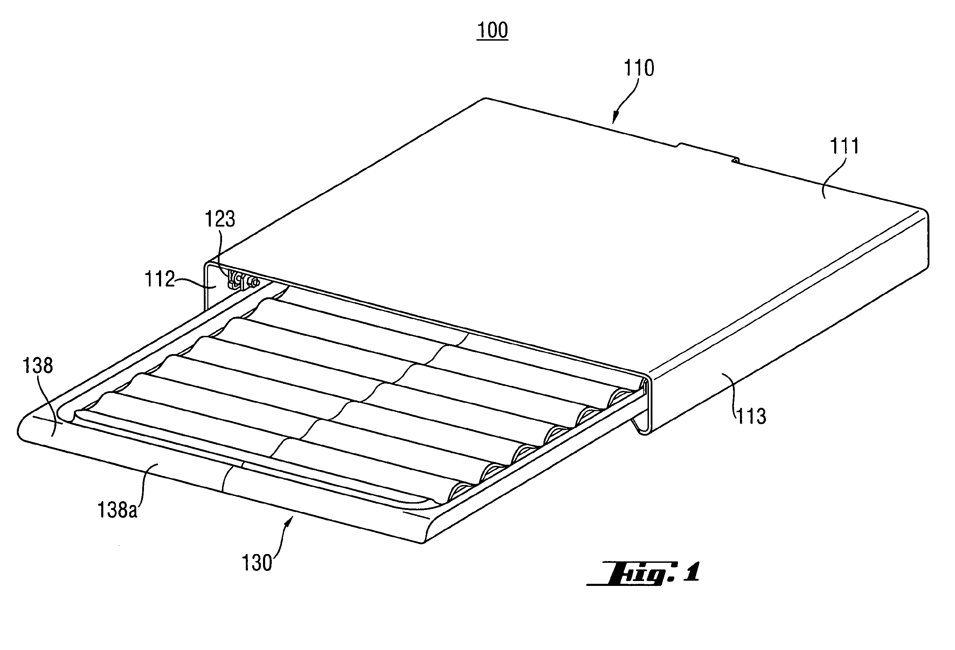

A right front perspective view of an inventive autoclave support base 100 is shown in FIG. 1. Support base 100 includes a base housing 110 and a holding shelf 130, the holding shelf 130 being slidably mounted in a cavity defined by a top surface 111, a left support member 112 and a right support member 113 of the base housing 110. Holding shelf 130 further includes a gripping surface 138a, which flows downwardly from a front edge 138 of holding shelf 130. Gripping surface 138a provides a convenient handle for extending and retracting holding shelf 130.

Base housing 110 and holding shelf 130 may each be formed in a conventional manner from a single piece of sheet steel. Alternatively, base housing 110 and holding shelf 130 may each be formed as conventional plastic moldings.

Although left and right support members 112, 113 are shown in FIG.1 as extending continuously along left and right edges of top surface 11, one skilled in the art will readily recognize that support members may be configured in a variety of alternative ways to support top surface 111. For example, support members 112, 113 may be alternatively configured as legs positioned at left and right or front and back edges of top surface 111. The legs may be positioned inwardly from rather than directly at the left and right or front and back edges.

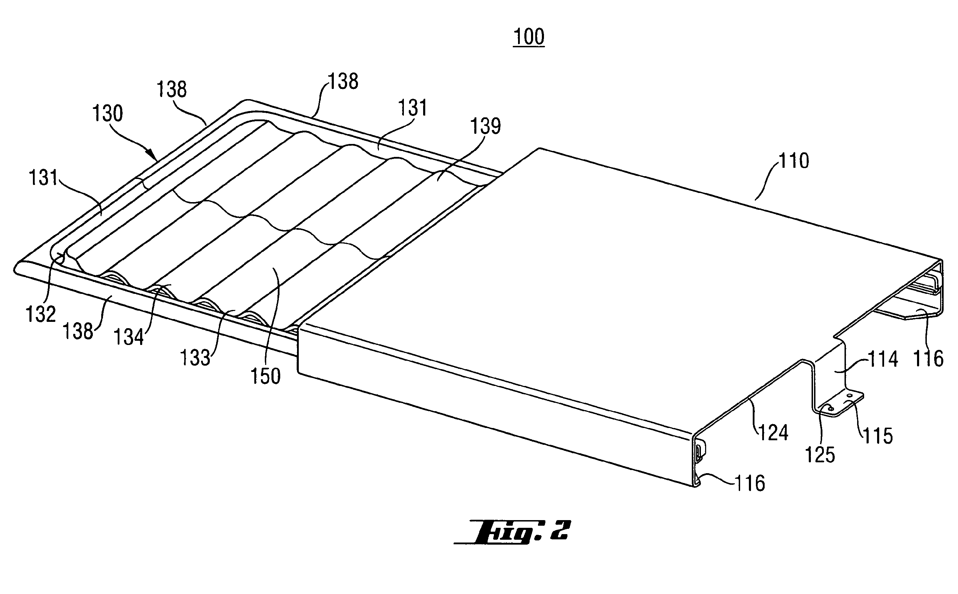

FIG. 2 shows a right rear perspective view of the support base 100 of FIG. 1. As illustrated in FIG. 2, base housing 110 further includes a rear support member 114 which is attached to a rear edge 124 of the base housing 110. Rear support member 114 is similar in shape and size to legs 112a, 113a earlier described. When placed upon a supporting surface (not shown), rear support member 114 provides additional rigidity to the top surface 111 of base housing 110. Optionally, rear support member 114 may further include a base flange 115, which together with support member flanges 116 provides an increased contact area between the base housing 110 and the supporting surface. Optionally, base flange 115 and support member flanges 116 may each contain one or more apertures 125 for fixedly attaching base housing 110 to the supporting surface using conventional fasteners.

Holding shelf 130 of FIG. 2 includes a holding surface 132 (partially obscured) which is depressedly positioned with respect to holding shelf edges 138. Surfaces 131 defined between edges 138 and holding surface 132 are approximately perpendicular to holding surface 132 and serve to confine items placed on holding surface 132 in proximity to one or more edges 138.

As shown in FIG. 2, holding shelf 130 may also optionally include a holding element 139, which is positioned near edges 138 and supported by holding surface 132. Holding element 139 may be fixedly fastened to holding surface 132 by any of a variety of fastening techniques, or alternatively, maybe integrally formed as part of holding shelf 130.

In FIG. 2, holding element 139 has a drying surface 150 which is further defined by a plurality of lateral troughs 133 and lateral crests 134. Items that have been sterilized in the autoclave, for example, may be positioned to rest across lateral crests 134 in order to promote air flow through lateral troughs 133 for improved drying and cooling of the sterilized items. Alternatively, lateral troughs 133 may be used to organize and hold some of the drying items (for example, such as dental picks and other slender instruments). Condensate that forms on the drying items may effectively drain into lateral troughs 133 to facilitate drying.

In order to more effectively remove condensate from the holding element 139, lateral troughs 133 may be pitched such that condensate drains to holding surface 132. Holding surface 132 may also be pitched so that this condensate may be led to a perforation in holding surface 132 for draining.

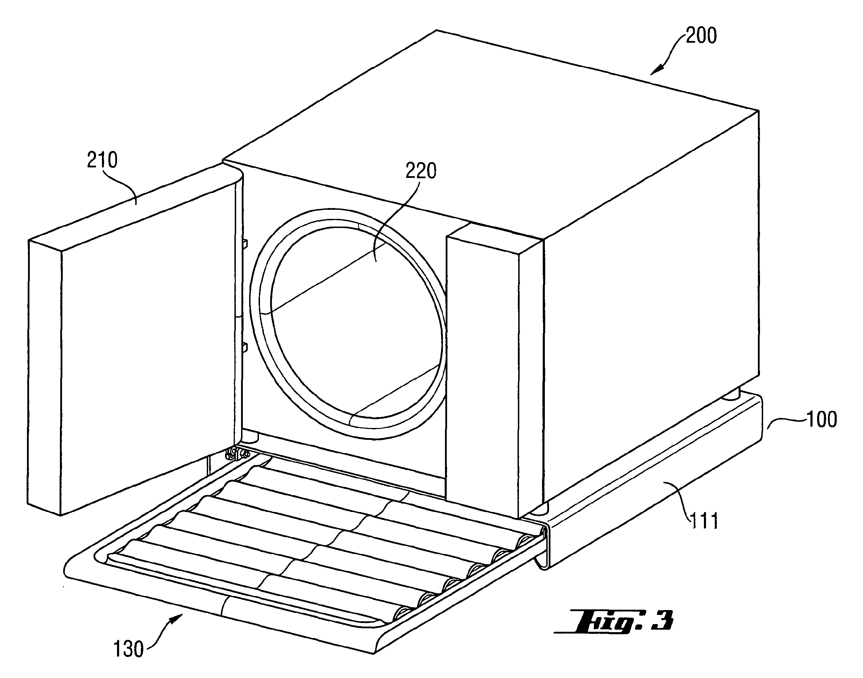

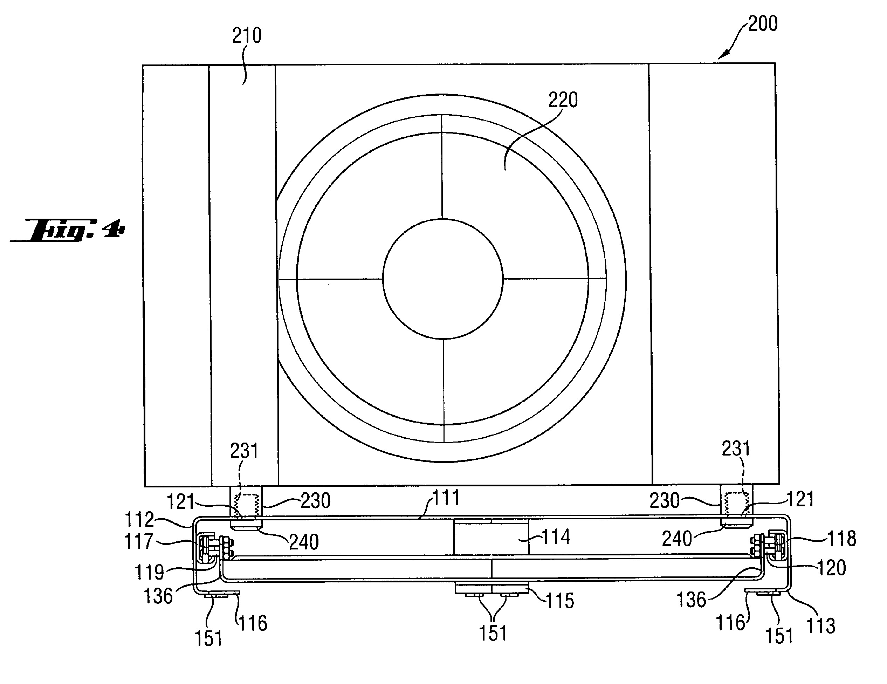

FIG. 3 illustrates an embodiment of the present invention in which top surface 111 of the support base 100 supports an autoclave 200. Autoclave 200 is, by way of example, a front-loading unit having a sterilizing chamber 220 and a sealing door 210. Holding shelf 130 of the support base 100 is conveniently positioned for loading items in and unloading items out of sterilizing chamber 220.

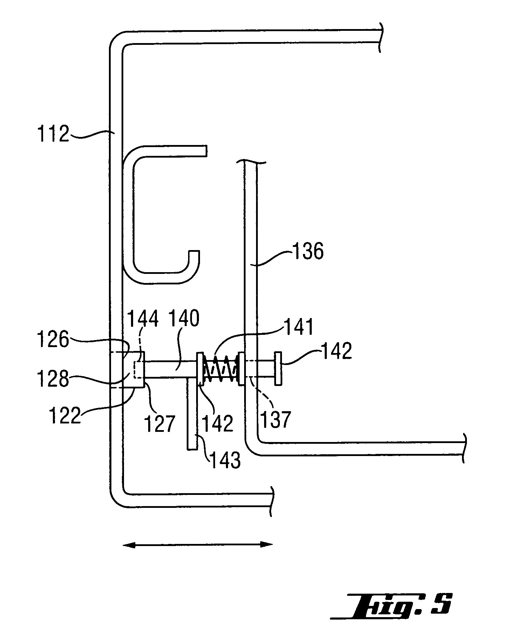

FIG. 4 provides a front view of the embodiment of FIG. 3. FIG. 4 illustrates conventional pull-out slides (sometimes referred to as drawer slides) that include a left track 117 fastened to left support member 112 and a right track 118 fastened to right support member 113. Glide wheel assemblies 119 and 120 are respectively retained by the tracks 117 and 118, which are fixedly attached to side surfaces 136 of holding shelf 130. Glide wheel assemblies 119 and 120 move within tracks 117 and 118 in order for holding shelf 130 to move from a retracted position mostly within the base housing 110 to an extended position mostly outside base housing 110. The extended position of holding shelf 130 is illustrated in FIGs. 1 - 3. As illustrated in FIG. 1, for example, retention stops 123 may be fitted at front ends of tracks 117 and 118 in order to prevent holding shelf 130 from being over-extended from base housing 110. Retention stops 123 (not shown) may also be optionally fitted at the rear ends of tracks 117 and 118 in order to more effectively position holding shelf 130 in the retracted position. Such track and glide assemblies are well-known in the art, and are readily available (for example, as part number C2132-20DOP from Accuride International Inc. of Sante Fe Springs, California).

FIG. 4 also illustrates how autoclave 200 may be secured to base housing 110. Autoclave 200 includes a plurality of feet 230, each of which contains a threaded insert 231. Top surface 111 of base housing 110 included a number of apertures 121 whose centerlines are positioned to align with centerlines of the threaded inserts 231 when autoclave 200 is appropriately positioned on top surface 111. Once positioned on top surface 111, autoclave 200 may be secured by inserting bolts 240 through the apertures 121 to mate with threaded inserts 231, and then appropriately tightening the bolts 240 until autoclave 200 is secured to top surface 111. Autoclave 200 may be secured to base housing 110 by a variety of other equivalent means. For example, tabs may be punched in top surface 111 to mate with slots in a front pair of autoclave feet 230, so that only a rear pair of autoclave feet 230 are fastened by inserting bolts 240 through a matching pair of rear apertures 121. Alternatively, lateral movement of autoclave 200 may be limited by providing indented or embossed regions in top surface 111 for restraining feet 230 of autoclave 200.

As earlier noted, base flange 115 and support member flanges 116 may each contain one or more apertures 125 for fixedly attaching base housing 110 to a supporting surface using conventional fasteners. Alternatively, and as illustrated in FIG. 4, base flange 115 and support member flanges 116 may be fitted with friction pads 151 to reduce lateral movement between flanges 115,116 and the supporting surface. Friction pads 151 may be adhesively or mechanically fastened to flanges 115,116, and may be fabricated from a variety of suitable materials including rubber and cork. As an additional alternative, flanges 115,116 may optionally be fitted with vertically-adjustable feet incorporating pads 151. Such adjustable feet are well-known in the art, and provide an advantage in stabilizing the autoclave unit on an uneven work surface.

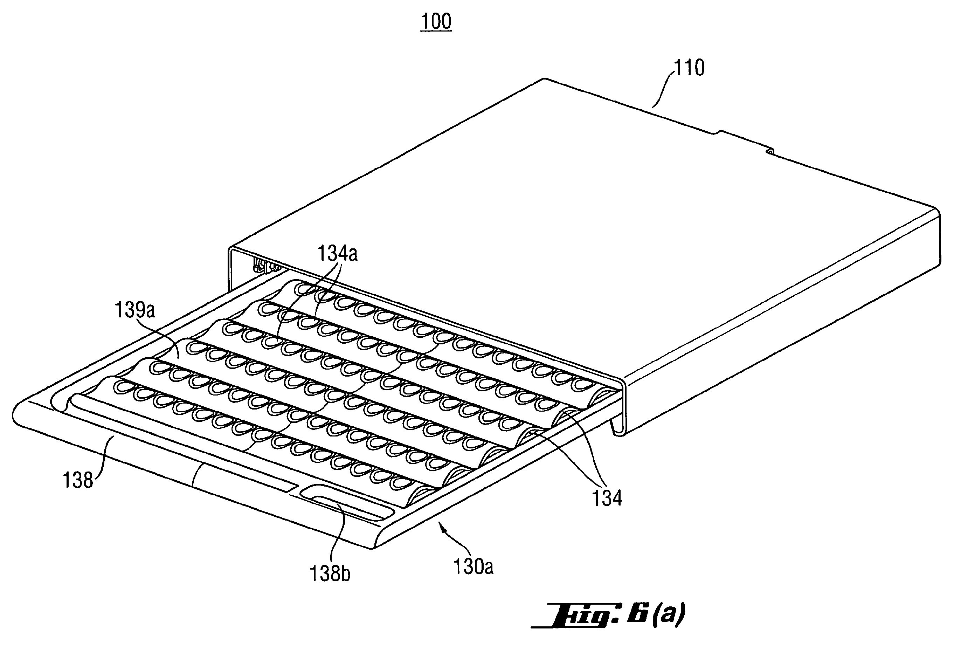

FIG. 5 presents an enlarged cross-sectional view taken near the front edge of support base 100 illustrated in FIG. 4. As shown in FIG. 5, a spring catch 140 is inserted through aperture 137 to be retained in side surface 136 of holding shelf 130 by retaining washers 142. Retaining washers 142 are affixed to spring catch 140 by any of a variety of conventional means.

When holding shelf 130 is retracted within base housing 110, force applied by spring 141 causes spring catch 140 to extend such that a distal end 144 of spring catch 140 is secured behind retaining tab 122 in left support member 112. In this position, holding shelf 130 is secured to remain in a retracted position within base housing 110. When it is desired to extract holding shelf 130, disengaging arm 143 may be manipulated to compress spring 141, moving spring catch 140 to a position where distal end 144 is no longer captive behind retaining tab 122. In this catch position, holding shelf 130 may be easily extracted from base housing 130.

It should be noted that retaining tab 122 may be, for example, punched from left support member 112 such that a front edge 126 of retaining tab 122 is coplanar with an inner surface of left support member 112, where front edge 126 is positioned outward from retaining edge 127. In this configuration, a cam surface 128 defined by edges 126, 127 acts to compress spring 141 and move catching spring 140 toward side surface 136 when force is applied to return holding shelf 130 to its retracted position. By such means, distal end 144 may be automatically moved behind retaining tab 122 without manually operating disengaging arm 143.

Various other mechanisms may be employed for retaining holding shelf 130 within base housing 110. For example, detents (not shown) may be incorporated in a lower portion of track 117, such that glide wheels from glide wheel assemblies 119 may be moved vertically against gravity force and horizontally rearward to rest behind and be retained by the detents. Retained glide wheels may then be released by moving the wheels vertically against gravity force and horizontally forward from the detents. Such gravity catches are well-known in the art, and are available, for example, in drawer slide part number D2132-20D from Accuride International Inc. Alternatively, conventional magnetic catches may be employed to secure holding shelf 130 in a retracted position within base housing 110. Catches may also be positioned to retain holding shelf 130 at one or more intermediate positions of extension from base housing 130.

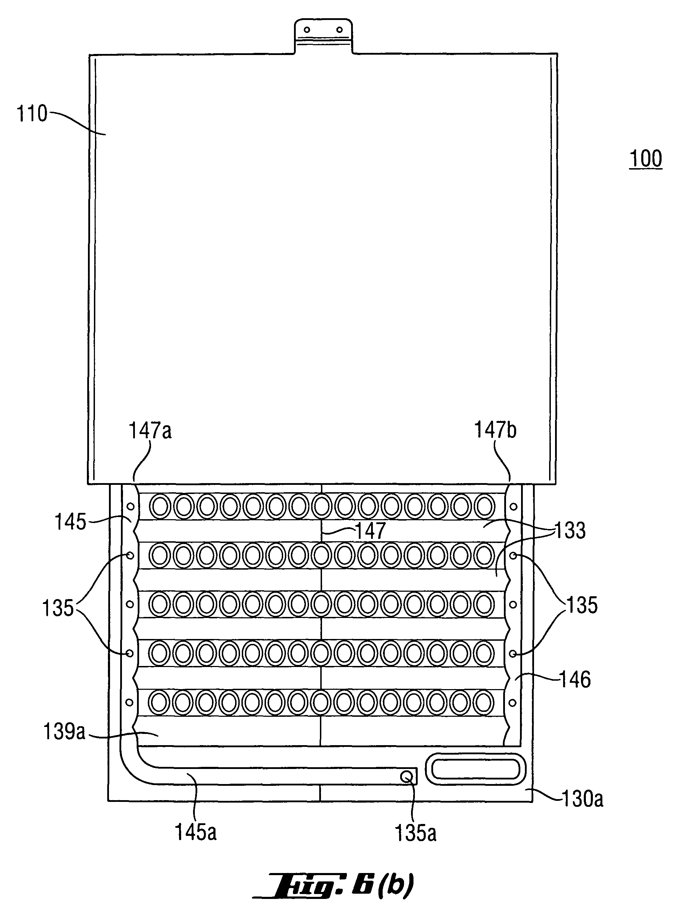

FIGs. 6(a), 6(b) illustrate an alternative holding element 139a and holding shelf 130a for support base 100. Holding element 139a of FIG. 6(a) is positioned in holding shelf 130a, which extends from base housing 110. Lateral crests 134 of holding element 139a are dimpled to include regularly-spaced indentations 134a. Indentations 134a provide a means for positioning items on crests 134 for drying. This means will be particularly useful, for example, in positioning thin, axially-oriented items such as dental instruments on the lateral crests.

As further illustrated in FIG. 6(a), holding shelf 130a includes cavity 138b near a right side of front edge 138. Cavity 138b may be used to hold small items not easily positioned on crests 134 for drying. Alternatively, cavity 138b may be used as an additional grip for extending and retracting holding shelf 130a.

Referring to FIGs. 2 , 6(a) and 6(b), holding elements 139, 139a may be fashioned from a variety of materials, including sheet steel and molded plastic, and may be either removably or fixedly mounted within holding shelves 130, 130a, respectively. Holding elements 139, 139a may also optionally be integrally formed with holding shelves 130, 130a.

FIG. 6(b) presents a top view of the holding element 139a positioned in holding shelf 130a of base housing 110. Perforations 135 are located along a bottom portion of channels 145, 146, respectively located at left and right edges147a, 147b of holding element 139a. Channels 145, 146 are positioned to receive condensate drainage from holding element 139a, which may be facilitated, for example, by pitching lateral troughs from centerline 147 downward towards channels 145, 146. Perforations 135 provide a means for draining condensate received by channels 145, 146.

Alternatively, in lieu of perforations 135, channel 145 may be pitched downwards toward perforation 135(a), which is positioned in channel extension 145a near a front edge of holding shelf 130a. This alternative configuration provides the advantage of positioning condensate drainage from holding shelf 130a near the front edge of the shelf where it can be more easily and confinedly dispersed. In this configuration, for example, channel 146 may be omitted, and lateral troughs 133 may be pitched downward from right edge 147b of holding element 139a toward channel 145.

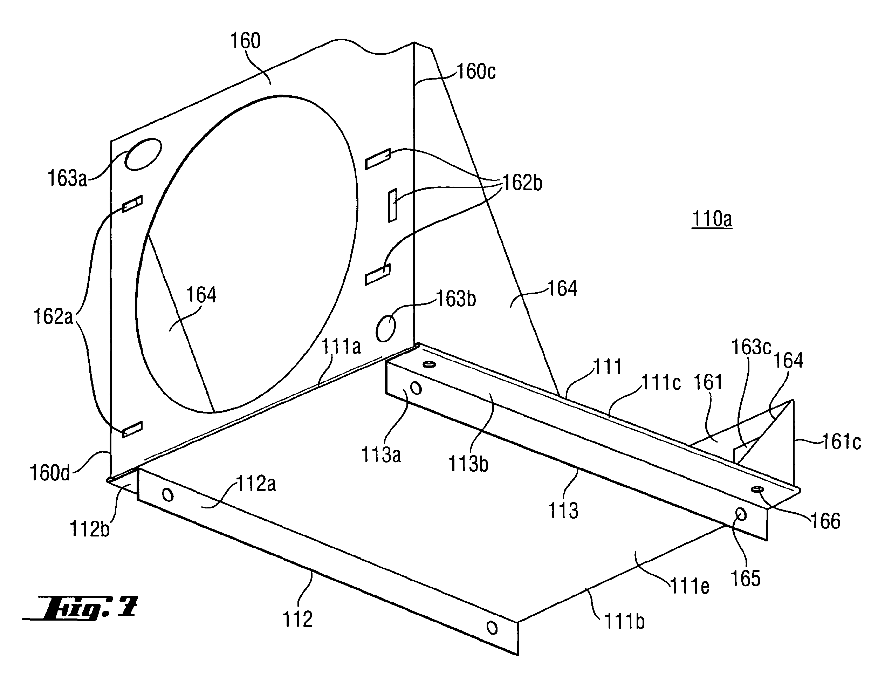

FIG. 7 presents a perspective view of a second base housing embodiment 110a. In base housing 110a, securing panels 160, 161 extend upwardly from top and rear edges 111a, 111b of top surface 111, respectively. Securing panels 160, 161 effectively define an internal chassis for autoclave 200. In other words, the internal and external components of autoclave 200 may be assembled to securing panels 160, 161 so that base housing 110a becomes an integral part of autoclave 200.

A illustrated in FIG. 7, front securing panel 160 includes autoclave chamber access aperture 162a to permit access to the autoclave chamber 220 of FIG. 2. In addition, hinge mount apertures 162a and latch mount apertures 162b respectively provide mounting points for hinge and latch hardware associated with autoclave 200. Access apertures 163a, 163b provide access for controls on the front panel of autoclave unit 200. Access aperture 163c may be provided on rear securing panel 161 for a similar purpose.

Gusset plates 164 are optionally attached at right and left edges 160c, 160d of front securing panel 160 and at right and left edges 111c, 111d of top surface 111 to make securing panel 160 more rigid. Gusset plates 164 may also be optionally attached at right and left edges 161c, 161d of rear securing panel 161 and right and left edges 111c, 111d of top surface 111. Although base housing 110a of FIG. 7 is illustrated with front securing panel 160 and rear securing panel 161, embodiments including only one of the front and rear panels 160, 161, as well as embodiments including one or more side panels attached at side edges 111c, 111d, are fully contemplated and within the scope of the present invention.

It should also be noted that, securing panels 160, 161 may be alternatively configured as an external chassis for confining autoclave 200 to top surface 111 of FIG. 7. In this alternative configuration, securing panels 160, 161 may be fastened to components affixed to the exterior of autoclave 200 (for example, fastening external hinge and latch hardware respectively through apertures 162a, 162b of securing panel 160.) In addition, gusset plates 164 and securing panels 160, 161 may be positioned in close proximity to exterior surfaces of autoclave 200 in order to restrict lateral movement of autoclave 200 on top surface 111. Other means (for example, as described in conjunction with FIG. 4) may be additionally employed to retain autoclave 200 against top surface 111.

FIG. 7 also illustrates an alternate embodiment for support members 112,113 of FIG. 1. In FIG. 7, support members 112a, 112b and support members 113a, 113b each form an L-shaped member 112,113 for supporting top surface 111. Members 112b,113b are respectively attached at edges 111d, 111c and to bottom surface 111e so that members 112a, 113a are vertically positioned with respect to top surface 111, and inwardly positioned with respect to edges 111d, 111c. Members 112a, 113a are also parallel to edges 111c, 111d , and to each other.

Apertures 165 in members 112a, 113a may be used to fasten tracks 117, 118 (not shown) on interior surfaces of members 112a, 113a. Alternatively, tracks 117,118 may be fastened to members 112a, 113a by a variety of other means including spot welding and gluing. Apertures 166 may be used for securing members 112, 113 to bottom surface 111e. Again, alternative fastening means such as spot welding and gluing may be used. Although members 112, 113 may simply be fastened to edges 111d, 111c, for example, as a folded construction from a single piece of sheet metal, it may be advantageous to provide additional fastening between members 112b, 113b and bottom surface 111e for added strength. If such additional fastening means are employed, apertures 166 may be alternatively used, for example, to mount adjustable feet for positioning base housing 110a on an uneven work surface. As depicted, base housing 110a may be fashioned from one or more pieces of folded sheet steel using conventional assembly methods. For strength and corrosion resistance, stainless steel is a preferred material for base housings 110, 110a, although molded plastics and other materials may be employed as well.

While the present invention has been described at some length and with some particularity with respect to the several described embodiments, it is not intended that it should be limited to any such particulars or embodiments or any particular embodiment, but it is to be construed with references to the appended claims so as to provide the broadest possible interpretation of such claims in view of the prior art and, therefore, to effectively encompass the intended scope of the invention.

## Claims

1. A sterilizer support base, the support base comprising:
a base housing, said base housing having a top surface for receiving and supporting a sterilizer unit, and left and right support members respectively and downwardly attached from the top surface, wherein the top surface and the left and right support members define a cavity in said base housing; and
a holding shelf, said holding shelf being slidably mounted within the cavity in said base housing.

2. The sterilizer support base of claim 1, wherein the base housing further comprises a rear support member downwardly attached from a rear edge of the top surface.

3. The sterilizer support base of claim 2, wherein each of the left, right and rear support member includes a base flange for making contact with a work surface.

4. The sterilizer support base of claim 1, wherein said holding shelf includes a holding surface depressedly positioned with respect to one or more edges of said holding shelf such that surfaces defined between said one or more edges of said holding shelf and said holding surface act to confine items placed on said holding surface.

5. The sterilizer support base of claim 4, wherein the holding shelf further includes a gripping surface, the gripping surface being located near a front edge of the holding shelf.

6. The sterilizer support base of claim 4, further comprising a holding element positioned near said one or more edges of said holding shelf and supported by said holding surface.

7. The sterilizer support base of claim 6, wherein a drying surface of the holding element comprises a plurality of lateral troughs alternatively spaced among a plurality of lateral crests, the plurality of lateral crests being arranged to hold items placed on the holding surface in an elevated position above the plurality of troughs.

8. The sterilizer support base of claim 7, wherein ones of the plurality of crests further have one or more indentations for positioning items on the plurality of crests.

9. The sterilizer support base of claim 8, wherein the one or more indentations are regularly spaced along ones of the plurality of crests.

10. The sterilizer support base of claim 6, wherein the holding element is fixedly attached to said holding shelf.

11. The sterilizer support base of claim 6, wherein the holding element is integrally formed with said holding shelf.

12. The sterilizer support base of claim 6, wherein the holding element is removably attached to said holding shelf.

13. The sterilizer support base of claim 6, wherein said holding shelf includes one or more perforations to facilitate drainage of condensate.

14. The sterilizer support base of claim 13, wherein said holding surface is pitched so that the condensate drains at the one or more perforations.

15. The sterilizer support base of claim 13, wherein one or more surfaces of the holding element are pitched.

16. The sterilizer support base of claim 1, wherein the holding shelf is slidably mounted to the base housing by slides respectively connecting the left and right support members to left and right edges of said holding shelf.

17. The sterilizer support base of claim 16, wherein the slides include one or more stops for limiting travel of said holding shelf in an extended position outside of the cavity.

18. The sterilizer support base of claim 16, wherein the base housing includes one or more stops for locating said holding shelf inside the cavity.

19. The sterilizer support base of claim 16, wherein said holding shelf further comprises a securing means for securing said holding shelf in at least one of a retracted position and an extended position with respect to the cavity.

20. The sterilizer support base of claim 19, wherein the securing means further secures said holding shelf at one or more predetermined intermediate positions of travel.

21. The sterilizer support base of claim 20, wherein the securing means includes means selected from the group consisting of mechanical and magnetic catches.

22. The sterilizer support base of claim 21, wherein the mechanical catch means operates with spring force.

23. The sterilizer support base of claim 21, wherein the mechanical catch means operates with gravity force.

24. The sterilizer support base of claim 3, wherein each of the left and right support member flanges extends inwardly from its respective support member with reference to the cavity.

25. The sterilizer support base of claim 1, wherein at least one of said base housing and said holding shelf is formed from a single piece of sheet steel.

26. The sterilizer support base of claim 1, wherein at least one of said base housing and said holding shelf is formed as a plastic molding.

27. The sterilizer support base of claim 6, wherein the holding element comprises a material from the group consisting of sheet steel and molded plastic.

28. The sterilizer support base of claim 7, wherein one or more of the lateral troughs is laterally pitched.

29. The sterilizer support base of claim 1, wherein sterilizer unit is fastened to the top surface of the base housing.

30. The sterilizer support base of claim 1, wherein the top surface includes one or more indented regions, each region for restrainably receiving a foot of the sterilizer unit.

31. The sterilizer support base of claim 2, wherein one or more of the left, right and rear support members comprises a means for securing the base housing to a work surface.

32. The sterilizer support base of claim 2, wherein one or more of the left, right and rear support members has one or more apertures.

33. The sterilizer unit of claim 32, wherein the apertures are each arranged to receive at least one of a fastener for mounting the base housing to a work surface and a foot for positioning the base housing on the work surface.

34. The sterilizer support base of claim 33, wherein the foot has a vertically-adjustable height.

35. The sterilizer support base of claim 1, the base housing further comprising
at least one securing panel, the at least one securing panel being upwardly attached at an edge of the top surface.

36. The sterilizer support base of claim 35, wherein the at least one securing panel is a component of the sterilizer unit.

37. The sterilizer support base of claim 35, wherein each of the left and right support members has an L-shaped cross section and a downwardly extending portion of each support member is positioned inwardly from the left and right opposing edges of the top surface.

38. The sterilizer support base of claim 35, wherein the at least one securing panel is arranged to be fixedly attachable to the sterilizer unit.

39. The sterilizer support base of claim 35, further comprising at least one gusset plate attached to a side edge of the at least one securing panel and a side edge of the top surface.

40. The sterilizer support base of claim 35, comprising at least two securing panels.

41. The sterilizer support base of claim 35, wherein one securing panel attached at the front edge of the top surface includes at least one aperture providing access to at least one of a sterilizing chamber and one or more controls of the sterilizer unit.

42. The sterilizer support base of claim 35, wherein the securing panel attached to the front edge of the top surface includes a plurality of apertures for accepting at least one of a door hinge mechanism and a door catch mechanism.

43. A retractable work surface for positioning in proximity to a sterilizer unit, the work surface comprising a plurality of lateral troughs alternatively spaced among a plurality of lateral crests, the plurality of lateral crests being arranged to hold items placed on the work surface in an elevated position above the plurality of troughs.

44. The retractable work surface of claim 43, further comprising slides for fixedly mounting the retractable work surface to the sterilizer unit.

45. The retractable work surface of claim 44, further comprising a retractable work surface base housing for holding the sterilizer unit, and wherein the slides are fixedly mounted to the base housing.

46. A sterilizer unit comprising a retractable work surface, the work surface including a plurality of lateral troughs alternatively spaced among a plurality of lateral crests, the plurality of lateral crests being arranged to hold items placed on the holding surface in an elevated position above the plurality of troughs.

47. The sterilizer support base of claim 29, wherein the top surface includes one or more apertures for receiving a fastener to secure the sterilizer unit to the base housing.
